(19) Europäisches Patentamt
European Patent Office
Office européen des brevets

(11) Veröffentlichungsnummer: **0 347 374 B1**

(12) **EUROPÄISCHE PATENTSCHRIFT**

(45) Veröffentlichungstag der Patentschrift: **15.12.93**

(51) Int. Cl.5: **C12P 7/42**, C12P 41/00, C07D 223/16

(21) Anmeldenummer: **89810403.9**

(22) Anmeldetag: **30.05.89**

# (54) Verfahren zur Herstellung von Hydroxysäuren.

(30) Priorität: **06.06.88 CH 2138/88**

(43) Veröffentlichungstag der Anmeldung:
**20.12.89 Patentblatt 89/51**

(45) Bekanntmachung des Hinweises auf die Patenterteilung:
**15.12.93 Patentblatt 93/50**

(84) Benannte Vertragsstaaten:
**AT BE CH DE ES FR GB GR IT LI LU NL SE**

(56) Entgegenhaltungen:
**EP-A- 0 000 560**
**EP-A- 0 024 547**
**EP-A- 0 206 993**

**CHEMICAL ABSTRACTS, Band 70, Nr. 15, 14. April 1969, Columbus, OH (US); B. DOLLAR, Seite 31, Nr. 64543g&NUM;**

**CHEMICAL ABSTRACTS, Band 86, Nr. 11, 14. März 1977, Columbus, OH (US); F. GOETZ et al., Seite 194, Nr. 67303v&NUM;**

(73) Patentinhaber: **CIBA-GEIGY AG**
**Klybeckstrasse 141**
**CH-4002 Basel(CH)**

(72) Erfinder: **Ghisalba, Oreste, Dr.**
**Eschenweg 3**
**CH-4153 Reinach(CH)**
Erfinder: **Gygax, Daniel, Dr.**
**Wulliweg 357**
**CH-4204 Himmelried(CH)**
Erfinder: **Lattmann, René, Dr.**
**Rottmannsbodenstrasse 133**
**CH-4102 Binningen(CH)**
Erfinder: **Schär, Hans-Peter, Dr.**
**Eggfluhweg 11**
**CH-4147 Aesch(CH)**
Erfinder: **Schmidt, Elke, Dr.**
**Konvikt-Strasse 10A**
**D-7800 Freiburg(DE)**
Erfinder: **Sedelmeier, Gottfried, Dr.**
**Erlenweg 11**
**D-7801 Schallstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft ein Verfahren zur Herstellung des R-Enantiomeren der 2-Hydroxy-4-phenylbuttersäure der Formel

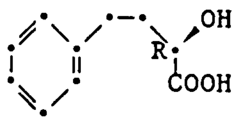

(I),

oder des S-Enantiomeren der 2-Hydroxy-4-phenylbuttersäure der Formel

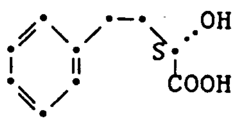

(II)

von sehr hoher Enantiomerenreinheit, z.B. im Bereich von 99 % ee (enantiomeric excess), bevorzugt von mehr als 99,6 % ee, dadurch gekennzeichnet, dass 2-Oxo-4-phenylbuttersäure mit dem Enzym D-Lactatdehydrogenase (D-LDH) aus Staphylococcus epidermidis bzw. mit dem Enzym L-Lactatdehydrogenase (L-LDH) aus Rinderherz in Gegenwart eines Elektronendonors, beispielsweise NAD(H), und eines Enzym-Substrat-Systems zur Regenerierung des Elektronendonors, beispielsweise Formiatdehydrogenase(FDH)-Formiat, reduziert wird. Bevorzugt ist das Verfahren zur Herstellung von R-2-Hydroxy-4-phenylbuttersäure der Formel I mit dem Enzym D-LDH aus Staphylococcus epidermidis in Gegenwart eines Elektronendonors, beispielsweise NAD(H), und eines Enzym-Substrat-Systems zur Regenerierung des Elektronendonors, beispielsweise Formiatdehydrogenase(FDH)-Formiat. Das erfindungsgemässe Verfahren eignet sich besonders gut für eine kontinuierliche enzymatische Umsetzung, bevorzugt in einem Enzym-Membranreaktor (EMR).

Die R-2-Hydroxy-4-phenylbuttersäure der Formel I ist ein wertvolles Zwischenprodukt bei der Herstellung von ACE (angiotensin converting enzyme)-Inhibitoren oder deren Vorstufen. Die S-2-Hydroxy-4-phenylbuttersäure der Formel II wird für die Herstellung isomerer Verbindungen eingesetzt.

Die Gewinnung chiraler Verbindungen durch stereospezifische mikrobiologische Reduktion ist bekannt (für einen Ueberblick vgl. Simon et al., Angew. Chemie 97, 541, 1985). Als Biokatalysatoren werden häufig intakte Mikroorganismen eingesetzt, etwa Pilze (z.B. Mucor, Geotrichum, Saccharomyces, Candida) oder Bakterien (z.B. Proteus, Pseudomonas). Es können auch mikrobielle Extrakte verwendet werden. Elektronendonoren sind beispielsweise Kohlenhydrate (z.B. Glucose), Formiat, Ethanol, Wasserstoff oder die Kathode einer elektrochemischen Zelle. Die Reduktion des Substrats erfolgt durch die sogenannte finale Reduktase, z.B. durch eine substratspezifische Dehydrogenase. Im allgemeinen werden die Reduktionsäquivalente, die von der finalen Reduktase benötigt werden, von einem Coenzym geliefert, z.B. von Pyridinnucleotiden wie NADH (Nicotinamidadenindinucleotid) und NADPH (Nicotinamidadenindinucleotidphosphat) oder von Flavinnucleotiden wie FMNH (Flavinmononucleotid) und FADH (Flavinadenindinucleotid). Die reduzierten Nucleotide ihrerseits entstehen üblicherweise in einer Serie von enzymkatalysierten Schritten unter Bildung konkurrierender Elektronenakzeptoren oder durch Elektronenübertragung durch natürliche oder synthetische Mediatoren (z.B. Ferredoxin, Viologene). Es sind auch finale Reduktasen bekannt, die Elektronen direkt von den Mediatoren aufnehmen können.

Als Biokatalysatoren eignen sich auch gereinigte Enzyme, d.h. isolierte Reduktasen, wobei im allgemeinen reduzierte Pyridin- bzw. Flavinnucleotide zugesetzt werden müssen. Ausserdem wird ein effizientes System zur enzymatischen Regeneration des Coenzyms benötigt, d.h. ein zweites Enzym und sein Substrat. Yamazaki & Maeda (Agricol. Biol. Chem. 50, 2621, 1986) beschreiben ein diskontinuierliches Verfahren zur Synthese von R-(-)-Mandelsäure aus Benzoylformiat mit Hilfe von NADH und der Benzoylformiat-Dehydrogenase aus Streptococcus faecalis. Dieses Verfahren kann auch kontinuierlich im Bioreaktor unter Regenerierung des Coenzyms mittels Formiatdehydrogenase und Formiat betrieben werden (Yamazaki & Maeda, Agricol. Biol. Chem. 50, 3213, 1986). Die Europäische Patentschrift EP O O24 547 beschreibt ein Verfahren zur kontinuierlichen enzymatischen Umwandlung von wasserlöslichen α-Ketocarbonsäuren in die entsprechenden α-Hydroxycarbonsäuren im Enzym-Membranreaktor. Die Umsetzung erfolgt in Gegenwart von durch Bindung an Polyethylenglykol molekulargewichtsvergrössertem NAD-

(H) und einer Lactatdehydrogenase unter gleichzeitiger NADH-Regenerierung durch Formiatdehydrogenase und Formiat.

Von ausschlaggebender Bedeutung bei enzymatischen Reaktionen sind die Eigenschaften und die Herkunft des eingesetzten Enzyms, in diesem Fall also der finalen Reduktase bzw. der substratspezifischen Dehydrogenase. Dabei muss im Auge behalten werden, dass sich auch gleichartige Enzyme in ihrem physiologischen Verhalten unterscheiden können, wenn sie aus unterschiedlichen Quellen, z.B. verschiedenen Mikroorganismen, isoliert wurden. Dabei variieren für die Biokonversion so entscheidende Parameter wie Reaktions-, Substrat- und Stereospezifität und kinetische Faktoren wie die Michaelis-Menten- und die Inhibitionskonstante (Simon et al., a.a.O.). Beispielsweise ergibt sich beim Vergleich der Daten aus der bekannten Literatur, dass die D-Lactatdehydrogenase aus Lactobacillus confusus zwar Pyruvat, 2-Ketobutyrat und Phenylpyruvat umsetzt, 2-Ketovalerat, 2-Ketocaproat und 2-Keto-3-methylvalerat von diesem Enzym jedoch nicht reduziert werden. Das Verhalten von einzelnen Enzym-Substrat-Systemen muss also jeweils getestet werden und lässt sich nicht verallgemeinernd voraussagen, auch wenn in EP O O24 547 dieser Schluss nahegelegt wird.

Aufgabe der vorliegenden Erfindung ist es, leistungsfähige Verfahren zur Herstellung des R- bzw. S-Enantiomeren von 2-Hydroxy-4-phenylbuttersäure in hoher Enantiomerenreinheit durch enantioselektive enzymatische Reduktion von 2-Oxo-4-phenylbuttersäure zu finden. Diese Substanz ist als Substrat im Stand der Technik der enzymatischen Reduktion von α-Ketocarbonsäuren nicht beschrieben und somit in Bezug auf ihre Eignung bzw. ihr Verhalten bei der enzymatischen Reduktion nicht untersucht.

Als besonders geeignet für die Lösung dieser Aufgabe erweist sich für die Herstellung von R-2-Hydroxy-4-phenylbuttersäure ein Verfahren, bei dem das Substrat mit dem Enzym D-Lactatdehydrogenase aus Staphylococcus epidermidis in Gegenwart eines Elektronendonors und eines Enzym-Substrat-Systems zur Regenerierung des Elektronendonors reduziert wird, da sich die D-LDH aus Staphylococcus epidermidis im Vergleich zu Lactatdehydrogenasen aus anderen Mikroorganismen vor allem durch eine hohe spezifische Aktivität (Einheit/mg umgesetztes Substrat bzw. μmol Umsatz/mg Protein x min.) in Bezug auf das eingesetzte Substrat auszeichnet (siehe Tab. 1) und eine hohe Enantioselektivität aufweist. Aus den gleichen Gründen ist für die Herstellung von S-2-Hydroxy-4-phenylbuttersäure ein analoges Verfahren besonders geeignet, bei dem das Substrat mit dem Enzym L-Lactatdehydrogenase aus Rinderherz reduziert wird. Für beide Verfahren ist die kontinuierliche Reaktionsführung, insbesondere in einem Enzym-Membranreaktor, bevorzugt.

Tabelle 1

| Vergleich der spezifischen Aktivität käuflicher Dehydrogenasen | | |
|---|---|---|
| Enzym Quelle | U/mg Protein bezogen auf Pyruvat | U/mg Protein bezogen auf 2-Keto-4-phenylbuttersäure |
| D-LDH Lactobacillus leichmannii Boehringer 732737 | 300 | ~0,1 |
| D-LDH Lactobacillus leichmannii Sigma L 2011 | 300 | 0,3 |
| D-LDH Leuconostoc mensenteroides Sigma L 0513 | 1000-1500 (1225) | 1,23 |
| D-LDH Staphylococcus epidermidis Sigma L 9636 | 500-1000 (625) | 26 |
| L-LDH Rinderherz Fluka 61310 | 300 | 0,07 |
| 700 U = 1 Mol Produkt/Tag | | |

Die Kombination der D-LDH aus Staphylococcus epidermidis als substratspezifische Dehydrogenase mit hoher Enantioselektivität und 2-Oxo-4-phenylbuttersäure als Substrat bietet die Gewähr für hohe Produktivitätszahlen, gute Raum-Zeit-Ausbeuten und damit im Zusammenhang stehend Preisgünstigkeit, was für enzymatische Umsetzungen im grosstechnischen Massstab von grosser Bedeutung und von erheblichem wirtschaftlichem Vorteil ist.

Als Elektronendonor für die D-Lactatdehydrogenase aus Staphylococcus epidermidis bzw. für die L-Lactatdehydrogenase aus Rinderherz wird vorzugsweise das Coenzym Nicotinamidadenindinucleotid in seiner reduzierten Form (NADH) verwendet, das durch die D- bzw. L-LDH zu NAD oxidiert wird. Zur Regenerierung des Coenzyms wird ein Enzym-Substrat-System eingesetzt, das aus einem NADH-recyclisierenden Enzym und seinem Substrat, z.B. Formiat, Ethanol, Isopropanol, Cyclohexanol u.a., besteht. Bevorzugt ist ein Formiatdehydrogenase(FDH)-Formiat-System, bei dem als Formiat ein Salz der Ameisensäure, beispielsweise ein Alkalimetallformiat, z.B. Kalium- bzw. Natriumformiat, verwendet wird, oder ein Alkoholdehydrogenase(ADH)-Ethanol-System. Dabei entstehen als Nebenprodukte $CO_2/HCO_3^-$ bzw. Acetal-

dehyd.

Das erfindungsgemässe Verfahren liefert das Produkt R- bzw. S-2-Hydroxy-4-phenylbuttersäure in hoher Enantiomerenreinheit. Im Zusammenhang mit der vorliegenden Beschreibung heisst "in hoher Enantiomerenreinheit", dass das entsprechende Enantiomere mit mindestens 98 % ee im Gemisch mit dem anderen Enantiomeren vorliegt, bevorzugt mit mehr als 99 % ee.

Im diskontinuierlichen (batch) Verfahren wird eine wässerige Lösung des Substrats 2-Oxo-4-phenylbuttersäure, beispielsweise in Form ihres Kalium- oder Natriumsalzes, in einer Konzentration von bis zu 500 mM, beispielsweise in einer Konzentration von 20 bis 200 mM, bevorzugt von 50 mM, mit dem Coenzym NAD(H) in einer Konzentration von 0,01 bis 10 mM, vorzugsweise von etwa 0,1 mM, dem NADH-recyclisierenden Enzym, z.B. einer Alkohol- oder einer Formiatdehydrogenase, und Formiat bzw. Ethanol in einer Konzentration von 100 bis 1200 mM, bevorzugt von etwa 300 mM, sowie der D-Lactatdehydrogenase aus Staphylococcus epidermidis bzw. der L-Lactatdehydrogenase aus Rinderherz bis zum Totalumsatz unter Rühren inkubiert. Die Enzyme werden zweckmässig in solchen Mengen eingesetzt, dass das Verhältnis der Aktivitäten von NADH-recyclisierendem Enzym und substratspezifischer Dehydrogenase zwischen 1:0,1 und 1:5 liegt. Das Reaktionsgemisch hat einen für enzymatische Reaktionen üblichen pH-Wert im Bereich zwischen pH 6 und 9, z.B. um pH 8,4. Die Reaktionstemperatur beträgt zwischen 20 °C und 40 °C, bevorzugt um Raumtemperatur. Das Produkt wird durch Zugabe von Säure, beispielsweise einer Mineralsäure wie Salzsäure u.a., aus dem Reaktionsgemisch auskristallisiert.

Für die kontinuierliche Reaktionsführung werden die eingesetzten Enzyme im allgemeinen immobilisiert. Sie können beispielsweise in polymere Hüllsubstanzen (Matrices), in Kapseln bzw. Fasern aus semipermeablen Membranen oder durch Ultrafiltrationsmembranen eingeschlossen, mit bi- bzw. multifunktionellen Reagenzien quervernetzt oder durch Adsorption, durch ionische oder kovalente Bindung an Träger aus anorganischem Material oder natürlichen bzw. synthetischen Polymeren fixiert sein. Es kann eine Vielzahl von Bioreaktortypen für den kontinuierlichen Prozess eingesetzt werden, z.B. Rühr-, Festbett-, Wirbelschicht- oder Membranreaktoren (für einen Ueberblick vgl. Hartmeier, "Immobilisierte Biokatalysatoren", Berlin 1986).

Beim erfindungsgemässen EMR-Verfahren (kontinuierliches Verfahren im Enzym-Membranreaktor) wird als Reaktionsgefäss bevorzugt ein Membranreaktor verwendet, der mit einer Ultrafiltrationsmembran ausgestattet ist, die die eingesetzten Enzyme und das für die Umsetzung erforderliche Coenzym zurückhält, das niedermolekulare Produkt und das nicht umgesetzte Substrat jedoch durchlässt. Ein wesentlicher Vorteil der Membranreaktoren liegt darin, dass die Biokatalysatoren nativ, d.h. nicht modifiziert, eingesetzt werden können und keinem der sonst zur Immobilisierung notwendigen, üblicherweise inaktivierend wirkenden Fixierungsschritte unterworfen werden müssen. Bei dem Enzym-Membranreaktor kann es sich beispielsweise um einen Flachmembran- (Kammermembran-) oder um einen Hohlfaser-Membranreaktor handeln. Das Substrat wird z.B. durch eine Dosierpumpe dem Reaktionsraum zugeführt, das Reaktionsgemisch wird gerührt bzw. umgepumpt, und der durch die Membran hindurchtretende Filtratstrom, der das Produkt enthält, wird abgeführt. Für das erfindungsgemässe Verfahren werden vorzugsweise Membranen mit einer nominellen Ausschlussgrenze zwischen 5'000 und 100'000 Dalton, z.B. zwischen 10'000 und 100'000 Dalton, verwendet. Geeignete Materialien für die Membranen sind etwa Acetylcellulosen, Polyamide, Polysulfone oder modifizierte Polyvinylalkohole. Um die Adsorption der an der Reaktion beteiligten Enzyme an die Membran zu verhindern, kann die Membran mit einem unspezifischen Protein, etwa Rinderserum-Albumin, vorbelegt werden.

Das Reaktionsgemisch im Membranreaktor enthält das NADH-recyclisierende Enzym, beispielsweise eine Alkoholdehydrogenase oder bevorzugt eine Formiatdehydrogenase, die D-Lactatdehydrogenase aus Staphylococcus epidermidis bzw. die L-Lactatdehydrogenase aus Rinderherz, und NAD(H). Das NADH-recyclisierende Enzym wird zweckmässig in einer solchen Menge eingesetzt, dass das Verhältnis der Aktivitäten von NADH-recyclisierendem Enzym und substratspezifischer Dehydrogenase zwischen 1:0,1 und 1:5 liegt. Das erforderliche Coenzym wird in Form von nicht molekulargewichtsvergössertem (nativem) NAD(H) in einer Konzentration von 0,01 bis 10 mM, vorzugsweise von etwa 0,1 mM, eingesetzt. Die Möglichkeit, dass beim erfindungsgemässen Verfahren auch im Enzym-Membranreaktor natives NAD(H) eingesetzt werden kann, bietet gegenüber dem in EP 0 024 547 beschriebenen Stand der Technik deutliche Vorteile. EP 0 024 547 schreibt die Verwendung von NAD(H) vor, das zur Molekulargewichtsvergrösserung an ein Polyethylenglykol gebunden ist. Durch diese Bindung kann es jedoch zu einem Aktivitätsverlust der Enzyme kommen. So wird zum Beispiel die D-Lactatdehydrogenase aus Staphylococcus epidermidis bei Verwendung von PEG-NAD(H) als Coenzym gegenüber nativem NAD(H) so stark in ihrer Aktivität eingeschränkt, dass der $V_{max}$-Wert, d.h. die maximale Reaktionsgeschwindigkeit, nur noch 2,6 Units/mg gegenüber 26 Units/mg für natives NAD(H) beträgt. Will man in einem kontinuierlichen Prozess unter Verwendung von PEG-NAD(H) adäquate Umsetzungsgeschwindigkeiten des Substrates erreichen, muss daher ca.

zehnmal soviel Enzym im EMR eingesetzt werden, wodurch sich die Produktionskosten stark erhöhen. Um beispielsweise NAD(H), das durch Bindung an ein Polyethylenglykol mit dem Molekulargewicht 20'000 molekulargewichtsvergrössert ist, hinter einer Ultrafiltrationsmembran effizient zurückzuhalten, darf die Membran eine maximale Ausschlussgrenze von 10'000 Dalton haben. Bei Einsatz von katalytischen Mengen von nativem NAD(H) im Substratstrom wird dagegen die Ausschlussgrenze der Membran nur von der Enzymgrösse bestimmt. Es können daher Membranen mit Ausschlussgrenzen von 5'000 bis 100'000 Dalton eingesetzt werden, so dass hoher Druck im Reaktor, der sich begrenzend auf die Laufzeit des Reaktors auswirkt, vermieden werden kann. Aufgrund des niedrigen Drucks bei Verwendung von nativem NAD(H) können ausserdem kleinere und damit weniger kostenaufwendige Membranflächen eingesetzt und höhere Durchflussraten erzielt werden. Auch bei Verwendung von nativem NAD(H) werden hohe Zyklenzahlen im Bereich von 500-2'000 erreicht, d.h. pro Molekül NAD(H) werden 500-2'000 Moleküle Hydroxysäure gebildet. Damit bietet das erfindungsgemässe Verfahren erhebliche wirtschaftliche Vorteile gegenüber dem in EP O O24 547 beschriebenen Verfahren.

Ausserdem wird dem Reaktor kontinuierlich eine wässerige Lösung von 2-Oxo-4-phenylbuttersäure, beispielsweise in Form ihres Kalium- oder Natriumsalzes, als Substrat zugeführt. Das Substrat soll in einer Konzentration von maximal 500 mM vorliegen; bevorzugt ist eine Konzentration im Bereich zwischen 20 bis 200 mM, insbesondere von etwa 50 mM. Ebenso wird kontinuierlich Formiat bzw. Ethanol in einer Konzentration zwischen 100 bis 1200 mM zudosiert, bevorzugt Formiat in einer Konzentration von etwa 300 mM.

Das Reaktionsgemisch hat einen für enzymatische Reaktionen üblichen pH-Wert im Bereich zwischen pH 6 und 9, z.B. um pH 8,4. Die Reaktionstemperatur beträgt zwischen 20° und 40°C, bevorzugt um Raumtemperatur.

Bevorzugt ist ein erfindungsgemässes Verfahren wie im vorhergehenden beschrieben, dadurch gekennzeichnet, dass die enzymatische Umsetzung in einem Enzym-Membranreaktor erfolgt,

a) der mit einer Ultrafiltrationsmembran ausgestattet ist, deren nominelle Ausschlussgrenze beispielsweise zwischen 5'000 und 100'000 Dalton, bevorzugt zwischen 10'000 und 100'000 Dalton, beträgt und die gegebenenfalls mit einem unspezifischen Protein, z.B. Rinderserum-Albumin, vorbelegt ist,

b) der ein Reaktionsgemisch enthält, das aus einer Lösung einer Formiatdehydrogenase oder einer Alkoholdehydrogenase, vorzugsweise einer Formiatdehydrogenase, der D-Lactatdehydrogenase aus Staphylococcus epidermidis bzw. der L-Lactatdehydrogenase aus Rinderherz und von Nicotinamidadenindinucleotid (NAD(H)) in einer Konzentration von z.B. 0,01 bis 1 mM, bevorzugt von etwa 0,1 mM, besteht,

c) dem kontinuierlich eine wässerige Lösung des Substrats 2-Oxo-4-phenylbuttersäure, beispielsweise in Form ihres Kalium- oder Natriumsalzes, in einer Konzentration von bis zu 500 mM, beispielsweise in einer Konzentration von 20 bis 200 mM, bevorzugt von etwa 50 mM, und von Formiat, beispielsweise Kalium- oder Natriumformiat, bzw. Ethanol, z.B. in einer Konzentration von 100 bis 1200 mM, bevorzugt von etwa 300 mM, zugeführt wird, und

d) in dem hinter der Membran kontinuierlich die gebildete Verbindung abgeführt wird.

R-2-Hydroxy-4-phenylbuttersäure ist ein wertvolles Zwischenprodukt bei der Herstellung von ACE-Inhibitoren oder deren Vorstufen. Diese Wirkstoffklasse hat in den vergangenen Jahren wachsendes Interesse gefunden. Sie erweitert das Potential der verfügbaren Antihypertensiva und damit die therapeutischen Möglichkeiten bei der Bekämpfung des Bluthochdrucks. Bei einer Reihe von wirksamen ACE-Inhibitoren ist das Strukturelement der Teilformel

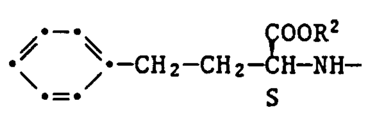 (III)

bedeutsam, worin $R^2$ für Wasserstoff oder Niederalkyl steht und welches in der S-Konfiguration vorliegt. R-2-Hydroxy-4-phenylbuttersäure lässt sich auf bekannte Weise und unter Erhalt der hohen Enantiomerenreinheit für die Herstellung von ACE-Hemmern einsetzen (siehe dazu z.B. die Europäische Patentanmeldung 206993). Der besondere Wert der vorliegenden Erfindung liegt unter anderem darin, dass bei der über zahlreiche Stufen verlaufenden Synthese von ACE-Hemmern bereits in einem relativ frühen Stadium der Synthese eine enantiomerenreine Verbindung eingesetzt werden kann. Von besonderem Interesse ist dabei die Herstellung des ACE-Hemmers 1-Carboxymethyl-3S-[(1S-ethoxycarbonyl-3-phenylpropyl)amino]-2,3,4,5-tetrahydro-1H-benzazepin-2-on. S-2-Hydroxy-4-phenylbuttersäure eignet sich in analoger Weise für die Herstellung von Enantiomeren des ACE-Hemmers sowie für Toxikologiestudien.

Die nachfolgenden Beispiele sollen die vorliegende Erfindung erläutern, ohne sie, etwa auf den Umfang der Beispiele, zu beschränken.

Abkürzungen

| | |
|---|---|
| ee | - Enantiomerenüberschuss ("enantiomeric excess") |
| FDH | - Formiatdehydrogenase |
| HPLC | - high pressure liquid chromatography |
| $K_I$ | - Inhibitionskonstante |
| $K_M$ | - Michaelis-Menten-Konstante |
| LDH | - Lactatdehydrogenase |
| NADH | - Nicotinamidadenindinucleotid |
| rpm | - Umdrehungen ("rotations") pro Minute |
| U | - Einheit ("unit") der Enzymaktivität (1 U bewirkt unter definierten Reaktionsbedingungen einen Stoffumsatz von 1 µmol/min) |
| $V_{max}$ | - maximale Reaktionsgeschwindigkeit |

Beispiel 1: Enzymatische Reduktion von 2-Oxo-4-phenylbuttersäure mit mikrobiellen Rohextrakten (Allgemeine Vorschrift)

Die Teststämme werden in 200 ml Nährlösung 148 (22 g/l Glucose, 5 g/l Lab-Lemco beef extract [Oxoid], 5 g/l Pepton C, 5 g/l Hefeextrakt, 3 g/l Bacto-Casein [Difco], 1,5 g/l NaCl, pH 6,5) oder Nährlösung MV7 (2 g/l $NH_4NO_3$, 1,4 g/l $Na_2HPO_4$, 0,6 g/l $K_2HPO_4$, 0,2 g/l $MgSO_4 \cdot 7H_2O$, 0,01 g/l $CaCl_2 \cdot 2H_2O$, 0,001 g/l $FeSO_4 \cdot 7H_2O$, 1 ml Spurenelement-Lösung [20 mg/l $Na_2MoO_4 \cdot 2H_2O$, 20 mg/l $Na_2B_4O_7 \cdot 10H_2O$, 20 mg/l $ZnSO_4 \cdot 7H_2O$, 20 mg/l $MnSO_4 \cdot H_2O$, 20 mg/l $CuSO_4 \cdot 5H_2O$), pH 6,5) mit 3 g/l D- bzw. L-Lactat 3 Tage bei 28°C unter Rühren (250 rpm) angezüchtet. Die Zellen werden mit Phosphatpuffer pH 7,0 gewaschen und durch Zentrifugation (20 min., 20000 rpm) in einer Sorvall-Zentrifuge, Rotor SS34, geerntet. Anschliessend werden die Zellen bei 4°C durch 45-minütige Ultraschallbehandlung bei 375 W in einem Ultrasonics Celldisrupter W-375 aufgeschlossen.

Nach erneuter Zentrifugation wird der Enzymrohextrakt mit dem Substrat in folgendem Testansatz für 3 - 5 Tage (bis zum Totalumsatz) bei 28°C unter Rühren inkubiert:
5 ml Zentrifugationsüberstand (Rohextrakt)
20 ml Phosphatpuffer pH 7 (0,069 M)
3 g/l 2-Oxo-4-phenylbuttersäure
18 g/l Ethanol
1 g/l NAD(H)
100 U Yeast Alcohol Dehydrogenase (Boehringer)
Nach beendeter Reaktion wird die Lösung mit 2N Salzsäure auf pH 2 eingestellt. Das dabei auskristallisierte Produkt wird mit Essigester extrahiert. Nach Abdestillieren des Lösungsmittels und Trocknen im Vakuum erhält man kristalline 2-Hydroxy-4-phenylbuttersäure unterschiedlicher Enantiomerenreinheit, abhängig vom getesteten mikrobiellen Extrakt.

Die kristalline Säure wird in absolutem Ethanol gelöst und mit Chlorwasserstoffgas während 24 Stunden bei Raumtemperatur zur Reaktion gebracht. Nach Abdestillieren des Alkohols und kurzem Entgasen im Hochvakuum verbleibt ein hellgelbes Oel, das an einer chiralen Säule (250 x 4,6 mm i.D., Durchfluss 1 ml/min, stationäre Phase Chiralcel OD [Stehelin, Basel] Typ OD-5-15-20925, mobile Phase: 90 % Hexan - 10 % Isopropanol - 0,1 % Diethylamin) durch HPLC bei 25°C/32 bar analysiert wird. Die zu analysierenden Substanzen liegen in einer Konzentration von 1 mg/ml im Fliessmittel vor (Einspritzmenge 10 µl). Das Scanning erfolgt bei einer Wellenlänge von 210 nm, die Auswertung durch Flächenvergleich mit externem Standard. Die ermittelten ee-Werte für die untersuchten mikrobiellen Extrakte sind in Tabelle 2 aufgeführt.

Tabelle 2

| Enantiomerenüberschuss bei der Reduktion mit mikrobiellen Rohextrakten | | |
|---|---|---|
| Teststamm (Extrakt) | ee | C-Quelle für Anzucht |
| Lactobacillus brevis DSM 20054 | 28 % (R) | Glucose |
| Staphylococcus epidermidis DSM 20042 | 78 % (R) | Glucose |
| Saccharomyces cerevisiae Backhefe Migros 76 10 2011 | 96 % (R) | Glucose |
| Kloeckera sp. 2201 ATCC 48 180 (Candida boidinii, T. Egli 2201) | 97 % (R) | Glucose |
| Saccharomyces cerevisiae Backhefe Migros 76 10 2011 | 90 % (R) | L-Lactat |
| Hansenula polymorpha CBS 4732 | 98 % (R) | D-Lactat |

Beispiel 2: Enzymatische Reduktion von 2-Oxo-4-phenylbuttersäure mit käuflichen Dehydrogenasen

Das Substrat wird mit einer käuflichen Dehydrogenase in folgendem Testansatz für 3 - 7 Tage (bis zum Totalumsatz) bei 28 °C unter leichtem Rühren inkubiert:
50 ml Phosphatpuffer pH 7 (0,069 M)
3 g/l 2-Oxo-4-phenylbuttersäure
18 g/l Ethanol
200 U Yeast Alcohol Dehydrogenase (Boehringer)
200 U Testenzym (käufliche Dehydrogenase)
1 g/l NAD(H)
Der Enantiomerenüberschuss an R-2-Hydroxy-4-phenylbuttersäure bzw. S-2-Hydroxy-4-phenylbuttersäure wird wie in Beispiel 1 bestimmt. Die Ergebnisse sind in Tabelle 3 zusammengefasst.

Tabelle 3

| Enantiomerenüberschuss bei der Umsetzung von 2-Oxo-4-phenylbuttersäure mit käuflichen Dehydrogenasen | |
|---|---|
| getestes Enzym | ee |
| D-LDH aus Lactobacillus leichmannii (Boehringer) | > 99 % (R) |
| D-LDH aus Lactobacillus leichmannii (Sigma) | > 98 % (R) |
| D-LDH aus Leuconostoc mesenteroides (Sigma) | > 98 % (R) |
| D-LDH aus Staphyloccoccus epidermidis (Sigma) | ~100 % (R)<br><O,2 % (S) |
| L-LDH aus Rinderherz (Fluka) | ~100 % (S) |

Ein Vergleich der ee-Werte zeigt, dass sich isolierte Enzyme für die stereospezifische Reduktion des Substrats besser eignen als mikrobielle Rohextrakte, da der Enantiomerenüberschuss an R- bzw. S-2-Hydroxy-4-phenylbuttersäure für isolierte Enzyme signifikant höher ist. Um ähnlich hohe ee-Werte zu erhalten, müssten die selektiven Enzyme aus den Rohextrakten noch durch Reinigungsschritte angereichert werden.

Beispiel 3: Enzymatische Reduktion von 2-Oxo-4-phenylbuttersäure mit käuflichen Dehydrogenasen im Enzym-Membranreaktor (EMR)

Die kontinuierliche Umsetzung von 2-Oxo-4-phenylbuttersäure zu R- bzw. S-2-Hydroxy-4-phenylbuttersäure erfolgt in einem auf 25 °C gehaltenen Flachmembran-Enzym-Membranreaktor (EMR) mit einem Reaktorvolumen von 10 ml. Die Celluloseacetat-Ultrafiltrationsmembran von 62 mm Durchmesser hat eine nominelle Ausschlussgrenze von 10'000 Dalton und ist mit 50 mg Rinderserum-Albumin vorbelegt.

Die optimale Reaktionsführung wird durch eine verfahrenstechnische Analyse mit Hilfe der experimentell ermittelten Enzymkinetik, d.h. durch Bestimmung der kinetischen Konstanten ($K_M$, $K_I$, $V_{max}$) für die D- bzw. L-Lactatdehydrogenase und die Formiatdehydrogenase jeweils für Substratkonzentrationen von 50, 100 und 150 mM ermittelt, indem durch Kalkulation der Massenbilanzen der Reaktanden das Verhalten des

Reaktors simuliert wird. Man wendet das "Runge-Kutta-Programm" an, bei dem die Parameter Verweilzeit, Edukt- und Cofaktorkonzentration und die Halbwertszeiten der Enzyme variiert werden (s. Hoffmann & Hoffmann, "Einführung in die Optimierung mit Anwendungsbeispielen aus dem Chemieingenieurwesen", Weinheim 1971).

Die Substratlösung enthält 50 mM 2-Oxo-4-phenylbuttersäure, 300 mM K-Formiat und O,1 mM NAD(H). Es werden 2,6 U/ml D-LDH und 4,8 U/ml FDH bzw. 1,4 U/ml L-LDH und 2,5 U/ml FDH eingespült. Die Reaktionslösung wird kontinuierlich mit 10 ml/h in den Reaktor gepumpt und das Produkt über die Membran abgeführt. Die Verweilzeit im Reaktor beträgt 60 Minuten für die Umsetzung mit D-LDH bzw. 120 Minuten für die Umsetzung mit L-LDH. Die Enzymaktivitäten werden laufend überwacht und gegebenenfalls durch Nachdosierung konstant gehalten.

Die Produktionsdaten sind in Tabelle 4 dargestellt.

Tabelle 4

| Produktionsdaten | | |
|---|---|---|
| | D-LDH | L-LDH |
| Versuchsdauer (kontinuierliche Produktion): | 450 h | 100 h |
| Umsatz: | ϕ 84 % | ϕ 77 % |
| Enantiomerenüberschuss: | ~ 100 % ee (R) | ~ 100 % ee (S) |
| Produktkonzentration: | ϕ 42,5 mM = 7,65 g/l | ϕ 38,5 mM = 6,8 g/l |
| Produktivität: | 184 g/l x d | 1,6 g/l x d |

Beispiel 4: Synthese von 1-Carboxymethyl-3S-[(1S-ethoxycarbonyl-3-phenylpropyl)-amino]-2,3,4,5-tetrahydro-1H-benzazepin-2-on (ACE-Inhibitor)

4.1. Synthese von R-2-Hydroxy-4-phenylbuttersäureethylester

5,0 g R-2-Hydroxy-4-phenylbuttersäure werden in 50 ml absolutem Ethanol gelöst und mit Chlorwasserstoff-Gas während 24 Stunden bei Raumtemperatur zur Reaktion gebracht. Nach Abdestillieren des Alkohols und kurzem Entgasen im Hochvakuum verbleibt ein hellgelbes Oel (5,7 g), welches laut HPLC-Analyse an einer chiralen Säule (s. Beispiel 1) zu ≧ 99,8 % aus dem R-konfigurierten Ester besteht. Der S-konfigurierte Ester ist zu weniger als 0,2 % vorhanden. Das Oel wird bei 100-105 °C und 6,5 Pascal destilliert und ergibt 5,2 g (-)-R-2-Hydroxy-4-phenylbuttersäureethylester mit einem Drehwert von $[\alpha]_D^{20}$ = -20,8 ° (1 % in Chloroform).

4.2. Synthese von (+)-R-2-(4-Nitrobenzolsulfonyloxy)-4-phenylbuttersäureethylester

9,75 g (46,8 mmol) (-)-R-2-Hydroxy-4-phenylbuttersäureethylester (≧ 99,6 % ee) werden in 50 ml Toluol gelöst, mit 11,4 g 4-Nitrobenzolsulfonylchlorid versetzt und anschliessend auf 0 °C gekühlt. Nach Zugabe von 6,25 g Triethylamin innerhalb von 30 Minuten wird auf Raumtemperatur erwärmt und aufgearbeitet. Man erhält in quantitativer Ausbeute (+)-R-2-(4-Nitrobenzolsulfonyloxy)-4-phenylbuttersäureethylester mit einem Drehwert von $[\alpha]_D^{20}$ = + 13,2 ° (3 % in abs. Ethanol).

4.3. Synthese von 1-Carboxymethyl-3S-[(1S-ethoxycarbonyl-3-phenylpropyl)amino]-2,3,4,5-tetrahydro-1H-benzazepin-2-on

46,1 g 3-(S)-Aminobenzazepin-2-on-1-N-essigsäure-tert.butylester, 84,3 g opt. reiner (≧ 99,6 % ee) (+)-R-2-(4-Nitrobenzolsulfonyloxy)-4-phenylbuttersäureethylester und 19,53 g N-Methylmorpholin werden ohne Lösungsmittel bei 75-80 °C 9 Stunden zur Reaktion gebracht. Das ausgefallene N-Methylmorpholinsalz der 4-Nitrobenzolsulfonsäure wird durch Zugabe von 250 ml Essigester und 150 ml Wasser in Lösung gebracht, mit ca. 150 ml 2 N Sodalösung auf pH 8,8 eingestellt, die Essigesterphase abgetrennt und noch zweimal mit Wasser gewaschen. Das nach Abdestillieren des Essigesters zurückbleibende Oel (98 g) zeigt im HPLC ein Diastereomerenverhältnis von mindestens SS : SR = 99,8 : 0,2.

Die Herstellung des Rohwirkstoffes erfolgt durch Einleiten von 54 g Chlorwasserstoffgas in eine Lösung von 96 g obigen Oels in 200 ml Essigester bei 0-10 °C. Nach vollständiger Solvolyse des tert.-Butylesters

fällt der Wirkstoff als feinkristalline Suspension an. Der überschüssige Chlorwasserstoff wird durch wiederholtes Abdestillieren von Essigester im Vakuum vollständig entfernt. Anschliessend wird die stark aufkonzentrierte Kristallsuspension mit 200 ml Aceton verdünnt, bei 15 °C abfiltriert und mit zweimal je 50 ml Essigester gewaschen. Nach Trocknen im Vakuum bei 60 °C bis zur Gewichtskonstanz werden 62,5 g (85,4 %) praktisch weisser Wirkstoff mit einem Diastereomerenverhältnis von SS : SR = 99,9 : 0,1 isoliert; $[\alpha]_D^{20}$ = - 138° (1 % in Ethanol abs.), Fp. 181 °C.

Beispiel 5: Synthese von 1-Carboxymethyl-3S-[(1R-ethoxycarbonyl-3-phenylpropyl)amino]-2,3,4,5-tetrayhdro-1H-benzazepin-2-on

Ausgehend von S-2-Hydroxy-4-phenylbuttersäure wird analog zu der in Beispiel 4 angegebenen Weise 1-Carboxymethyl-3S-[(1R-ethoxycarbonyl-3-phenyl-propyl)amino]-2,3,4,5-tetrayhdro-1H-benzazepin-2-on hergestellt.

**Patentansprüche**

1. Verfahren zur Herstellung des R-Enantiomeren der 2-Hydroxy-4-phenylbuttersäure der Formel

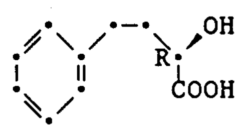

(I)

oder des S-Enantiomeren der 2-Hydroxy-4-phenylbuttersäure der Formel

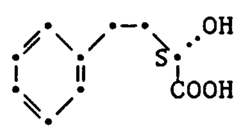

(II),

dadurch gekennzeichnet, dass 2-Oxo-4-phenylbuttersäure mit dem Enzym D-Lactatdehydrogenase (D-LDH) aus Staphylococcus epidermidis bzw. mit dem Enzym L-Lactatdehydrogenase (L-LDH) aus Rinderherz in Gegenwart eines Elektronendonors und eines Enzym-Substrat-Systems zur Regenerierung des Elektronendonors reduziert wird.

2. Verfahren nach Anspruch 1 zur Herstellung von R-2-Hydroxy-4-phenylbuttersäure, dadurch gekennzeichnet, dass 2-Oxo-4-phenylbuttersäure mit dem Enzym D-Lactatdehydrogenase (D-LDH) aus Staphylococcus epidermidis in Gegenwart eines Elektronendonors und eines Enzym-Substrat-Systems zur Regenerierung des Elektronendonors reduziert wird.

3. Verfahren nach Anspruch 1 oder 2, dadurch gekennzeichnet, dass man 2-Hydroxy-4-phenylbuttersäure in einer Enantiomerenreinheit von mehr als 99,6 % ee (enantiomeric excess) herstellt.

4. Verfahren nach Anspruch 1, 2 oder 3, dadurch gekennzeichnet, dass als Elektronendonor Nicotinamidadenindinucleotid (NAD(H)) und als Enzym-Substrat-System zur Regenerierung des Elektronendonors Formiatdehydrogenase(FDH)-Formiat eingesetzt wird.

5. Verfahren nach Anspruch 1, 2 oder 3, dadurch gekennzeichnet, dass als Elektronendonor Nicotinamidadenindinucleotid (NAD(H)) und als Enzym-Substrat-System zur Regenerierung des Elektronendonors Alkoholdehydrogenase(ADH)-Ethanol eingesetzt wird.

6. Verfahren nach einem der Ansprüche 1-5, dadurch gekennzeichnet, dass die enzymatische Umsetzung kontinuierlich erfolgt.

7. Verfahren nach einem der Ansprüche 1-6, dadurch gekennzeichnet, dass die enzymatische Umsetzung in einem Enzym-Membranreaktor erfolgt.

**8.** Verfahren nach Anspruch 7, dadurch gekennzeichnet, dass die enzymatische Umsetzung in einem Enzym-Membranreaktor erfolgt,
a) der mit einer Ultrafiltrationsmembran ausgestattet ist,
b) der ein Reaktionsgemisch enthält, das aus einer Lösung einer Formiatdehydrogenase oder einer Alkoholdehydrogenase, der D-Lactatdehydrogenase aus Staphylococcus epidermidis bzw. der L-Lactatdehydrogenase aus Rinderherz und von Nicotinamidadenindinucleotid (NAD(H)) besteht,
c) dem kontinuierlich eine wässerige Lösung des Substrats 2-Oxo-4-phenylbuttersäure und von Formiat bzw. Ethanol zugeführt wird, und
d) in dem hinter der Membran kontinuierlich die gebildete Verbindung abgeführt wird.

**9.** Verfahren nach Anspruch 7 oder 8, dadurch gekennzeichnet, dass die enzymatische Umsetzung in einem Enzym-Membranreaktor erfolgt,
a) der mit einer Ultrafiltrationsmembran mit einer nominellen Ausschlussgrenze zwischen 5'000 und 100'000 Dalton ausgestattet ist,
b) der ein Reaktionsgemisch enthält, das aus einer Lösung einer Formiatdehydrogenase oder einer Alkoholdehydrogenase, der D-Lactatdehydrogenase aus Staphylococcus epidermidis bzw. der L-Lactatdehydrogenase aus Rinderherz und von 0,01 bis 1 mM Nicotinamidadenindinucleotid (NAD(H)-) besteht,
c) dem kontinuierlich eine wässerige Lösung von bis zu 500 mM des Substrats 2-Oxo-4-phenylbuttersäure und von 100 bis 1200 mM Formiat bzw. Ethanol zugeführt wird, und
d) in dem hinter der Membran kontinuierlich die gebildete Verbindung abgeführt wird.

**10.** Verfahren nach einem der Ansprüche 7-9, dadurch gekennzeichnet, dass die enzymatische Umsetzung in einem Enzym-Membranreaktor erfolgt, dessen Ultrafiltrationsmembran mit einem unspezifischen Protein vorbelegt ist.

**Claims**

**1.** A process for the preparation of the R-enantiomer of 2-hydroxy-4-phenylbutyric acid of the formula

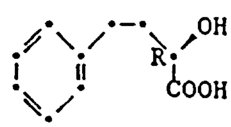

(I)

or the S-enantiomer of 2-hydroxy-4-phenylbutyric acid of the formula

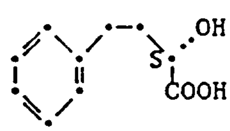

(II),

which process comprises reducing 2-oxo-4-phenylbutyric acid with the enzyme D-lactate dehydrogenase (D-LDH) from Staphylococcus epidermidis or with the enzyme L-lactate dehydrogenase (L-LDH) from bovine heart, respectively, in the presence of an electron donor and an enzyme/substrate system for regenerating the electron donor.

**2.** A process according to claim 1 for the preparation of R-2-hydroxy-4-phenylbutyric acid, which comprises reducing 2-oxo-4-phenylbutyric acid with the enzyme D-lactate dehydrogenase (D-LDH) from Staphylococcus epidermidis in the presence of an electron donor and an enzyme/substrate system for regenerating the electron donor.

**3.** A process according to claim 1 or 2, which comprises preparing 2-hydroxy-4-phenylbutyric acid in an enantiomeric purity of more than 99.6 % ee (enantiomeric excess).

4. A process according to claim 1, 2 or 3, which comprises using nicotinamide adenine dinucleotide (NAD(H)) as the electron donor and formate dehydrogenase (FDH)/-formate as the enzyme/substrate system for regenerating the electron donor.

5. A process according to claim 1, 2 or 3, which comprises using nicotinamide adenine dinucleotide (NAD(H)) as the electron donor and alcohol dehydrogenase (ADH)/-ethanol as the enzyme/substrate system for regenerating the electron donor.

6. A process according to any one of claims 1 to 5, which comprises carrying out the enzymatic conversion continuously.

7. A process according to any one of claims 1 to 6, which comprises carrying out the enzymatic conversion in an enzyme membrane reactor.

8. A process according to claim 7, which comprises carrying out the enzymatic conversion in an enzyme membrane reactor
   a) that is equipped with an ultrafiltration membrane,
   b) that contains a reaction mixture comprising a solution of a formate dehydrogenase or an alcohol dehydrogenase, the D-lactate dehydrogenase from Staphylococcus epidermidis or the L-lactate dehydrogenase from bovine heart, and nicotinamide adenine dinucleotide (NAD(H)),
   c) to which there is continuously fed an aqueous solution of the substrate 2-oxo-4-phenylbutyric acid and formate or ethanol, respectively, and
   d) in which the compound formed is continuously drawn off downstream of the membrane.

9. A process according to claim 7 or 8, which comprises carrying out the enzymatic conversion in an enzyme membrane reactor
   a) that is equipped with an ultrafiltration membrane having a nominal exclusion limit of from 5 000 to 100 000 daltons,
   b) that contains a reaction mixture comprising a solution of a formate dehydrogenase or an alcohol dehydrogenase, the D-lactate dehydrogenase from Staphylococcus epidermidis or the L-lactate dehydrogenase from bovine heart, and from 0.01 to 1 mM of nicotinamlde adenine dinucleotide (NAD(H)),
   c) to which there is continuously fed an aqueous solution of up to 500 mM of the substrate 2-oxo-4-phenylbutyric acid, and from 100 to 1200 mM of formate or ethanol, respectively, and
   d) in which the compound formed is continuously drawn off downstream of the membrane.

10. A process according to any one of claims 7 to 9 which comprises carrying out the enzymatic conversion in an enzyme membrane reactor of which the ultrafiltration membrane has been pre-coated with a non-specific protein.

## Revendications

1. Procédé pour la préparation de l'énantiomère R de l'acide 2-hydroxy-4-phénylbutyrique de formule

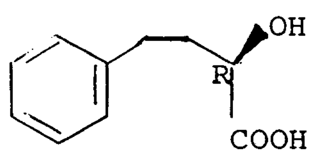

(I)

ou de l'énantiomère S de l'acide 2-hydroxy-4-phénylbutyrique de formule

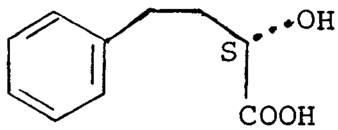

(II),

caractérisé en ce que l'on réduit l'acide 2-oxo-4-phénylbutyrique avec l'enzyme D-lactate-déshydrogénase (D-LDH) provenant du Staphylococcus epidermis ou avec l'enzyme L-lactate-déshydrogénase (L-LDH) provenant du coeur de boeuf en présence d'un donneur d'électrons et d'un système enzyme-substrat pour la régénération du donneur d'électrons.

**2.** Procédé selon la revendication 1 pour la, préparation de l'acide R-2-hydroxy-4-phénylbutyrique, caractérisé en ce que l'on réduit l'acide 2-oxo-4-phényl) butyrique avec l'enzyme D-lactate-déshydrogénase (D-LDH) provenant du Staphylococcus epidermis en présence d'un donneur d'électrons et d'un système enzyme-substrat pour la régénération du donneur d'électrons.

**3.** Procédé selon la revendication 1 ou 2, caractérisé en ce que l'on prépare l'acide 2-hydroxy-4-phénylbutyrique avec une pureté énantiomère supérieure à 99,6 % ee (enantiomeric excess).

**4.** Procédé selon les revendications 1, 2 ou 3, caractérisé en ce que l'on utilise, comme donneur d'électrons, le nicotinamide-adénine-dinucléotide (NAD(H)) et, comme système enzyme-substrat pour la régénération du donneur d'électrons la formiate-déshydrogénase formiate (FDH).

**5.** Procédé selon les revendications 1, 2 ou 3, caractérisé en ce que l'on utilise, comme donneur d'électrons, le nicotinamide-adénine-dinucléotide (NAD(H)) et, comme système enzyme-substrat pour la régénération du donneur d'électrons l'alcool-déshydrogénase (ADH)-éthanol.

**6.** Procédé selon l'une des revendications 1 à 5, caractérisé en ce que la transformation enzymatique s'effectue de façon continue.

**7.** Procédé selon l'une des revendications 1 à 6, caractérisé en ce que la transformation enzymatique s'éffectue dans un réacteur à membrane enzymatique.

**8.** Procédé selon la revendication 7, caractérisé en ce que la transformation enzymatique s'effectue dans un réacteur à membrane enzymatique,
a) équipé d'une membrane d'ultrafiltration,
b) contenant un mélange réactionnel constitué d'une solution d'une formiate-déshydrogénase ou d'une alcool-déshydrogénase, de D-lactate-déshydrogénase provenant du Staphylococcus epidermis ou de L-lactate-déshydrogénase provenant du coeur de boeuf et de nicotinamide-adénine-dinucléotide (NAD(H)),
c) dans lequel on amène, de façon continue, une solution aqueuse du substrat l'acide 2-oxo-4-phénylbutyrique et de formiate ou d'éthanol et
d) dans lequel on évacue, de façon continue, après la membrane le composé formé.

**9.** Procédé selon la revendication 7 ou 8, caractérisé en ce que la transformation enzymatique s'effectue dans un réacteur à membrane enzymatique
a) équipé d'une membrane d'ultrafiltration ayant une limite d'exclusion théorique comprise entre 5000 et 100 000 Daltons,
b) contenant un mélange réactionnel constitué d'une solution d'une formiate-déshydrogénase ou d'une alcool-déshydrogénase, de D-lactate-déshydrogénase provenant du Staphylococcus epidermis ou de L-lactate-déshydrogénase provenant du coeur de boeuf et de 0,01 à 1 mM de nicotinamide-adénone-dinucléotide (NAD(H)),
c) dans lequel on amène, de façon continue, une solution aqueuse contenant jusqu'à 500 mM du substrat l'acide 2-oxo-4-phénylbutyrique et de 100 à 1200 mM de formiate ou d'éthanol et
d) dans lequel on évacue, des façon continue, après la membrane le composé formé.

**10.** Procédé selon l'une des revendications 7 à 9, caractérisé en ce que la transformation enzymatique est effectuée dans un réacteur à membrane enzymatique dont la membrane d'ultrafiltration est préalablement chargée avec une protéine non spécifique.